(19)

**Europäisches Patentamt**
**European Patent Office**
**Office européen des brevets**

(11) **EP 2 605 774 B1**

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(51) Int Cl.:
*A61K 49/10* (2006.01)      *A61K 31/409* (2006.01)
*C07D 487/22* (2006.01)      *C09K 9/02* (2006.01)

(21) Anmeldenummer: **11784924.0**

(22) Anmeldetag: **10.08.2011**

(86) Internationale Anmeldenummer:
**PCT/DE2011/001573**

(87) Internationale Veröffentlichungsnummer:
**WO 2012/022299 (23.02.2012 Gazette 2012/08)**

(54) **MOLEKULARER SCHALTER**

MOLECULAR SWITCH

COMMUTATEUR MOLÉCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.08.2010 DE 102010034496**

(43) Veröffentlichungstag der Anmeldung:
**26.06.2013 Patentblatt 2013/26**

(73) Patentinhaber:
• **Christian-Albrechts-Universität zu Kiel**
  **24118 Kiel (DE)**
• **Universitätsklinikum Schleswig-Holstein**
  **24105 Kiel (DE)**

(72) Erfinder:
• **HERGES, Rainer**
  **24119 Kronshagen (DE)**
• **JANSEN, Olav**
  **24229 Strande (DE)**
• **TUCZEK, Felix**
  **24146 Kiel (DE)**
• **VENKATAMARANI, Sugumar**
  **24118 Kiel (DE)**

(74) Vertreter: **Hermann, Felix**
  **Boehmert & Boehmert**
  **Anwaltspartnerschaft mbB**
  **Pettenkoferstrasse 22**
  **80336 München (DE)**

(56) Entgegenhaltungen:

DE-A1- 10 039 903

• Bornholdt C: "Ligandgetriebener lichtinduzierter Spin-Crossover in Einzelmolekülen bei Raumtemperatur. Dissertation zur Erlangung des Doktorgrades der Mathematisch-Naturwissenschaftlichen Fakultät der Christian-Albrechts-Universität zu Kiel", 2008, Christian-Albrechts-Universität, Kiel, XP002667555, Seiten FP-244, in der Anmeldung erwähnt Seite 11, Zeile 1 - Seite 14, letzte Zeile Kapitel 5
• BOILLOT M-L ET AL: "FIRST LIGAND-DRIVEN LIGHT-INDUCED SPIN CHANGE AT ROOM TEMPERATURE IN A TRANSITION-METAL MOLECULAR COMPOUND", NEW JOURNAL OF CHEMISTRY, Bd. 23, Nr. 2, 1. Januar 1999 (1999-01-01) , Seiten 179-183, XP009005598, ROYAL SOCIETY OF CHEMISTRY, GB ISSN: 1144-0546, DOI: 10.1039/A809504C
• SOURA ET AL: "First Evidence of a Photoinduced Spin Change in an FeIII Complex Using Visible Light at Room Temperature", EUR. J. INORG. CHEM., Bd. 1999, Nr. 12, 15. November 1999 (1999-11-15), Seiten 2117-2119, XP002667556,
• MATINO F ET AL: "Single azopyridine-substituted porphyrin molecules for configurational and electronic switching", CHEMICAL COMMUNICATIONS, Bd. 46, Nr. 36, 28. September 2010 (2010-09-28), Seiten 6780-6782, XP002667557, ROYAL SOCIETY OF CHEMISTRY GBR ISSN: 1359-7345, DOI: 10.1039/C0CC00959H

EP 2 605 774 B1

# Beschreibung

Die Erfindung betrifft einen photosensitiven molekularen Schalter.

Insbesondere betrifft die Erfindung Komplexe von Übergangsmetallen, die über ein spezielles Ligandensystem verfügen, welches einen Wechsel der magnetischen Spinzustände ermöglicht, deren Herstellung sowie deren Anwendung u.a. als Kontrastmittel in der Magnetresonanztomographie als optisches Speichermedium und bei der berührungslosen Bewegung von Objekten.

In der US2007/0218010A1 und der EP 2 053 049 A1 werden Kontrastmittel für die Magnetresonanztomographie beschrieben, die über Liganden verfügen, die einen Wechsel des Spinzustandes am Zentralatom ermöglichen. Änderungen in der Koordinationssphäre durch enzymatische Abspaltung von Liganden (US2007/0218010A1) oder Änderungen in der Temperatur (EP 2 053 049 A1) oder pH-Wert können zu einer Änderung des magnetischen Zustandes führen (Spin-Crossover SCO) und damit zu einer Änderung der Wirkung als Kontrastmittel.

Das Vorgehen ist im Allgemeinen so, dass zunächst das Kontrastmittel einmal gespritzt wird und sich dann über den Blutkreislauf auf den ganzen Körper verteilt. Die Kontrastwirkung beruht darauf, dass es eher wasser- als fettlöslich ist. D.h. es reichert sich vor allem im Blut an und weniger im Fettgewebe. Das führt dazu, dass man die Blutgefässe sehr gut abbilden kann. Dieses Verfahren nennt man Magnetresonanz-Angiographie (MRA).

Für viele Anwendungen, z.B. bei der Untersuchung von Durchblutungsstörungen, vor allem bei der Darstellung der Herzkranzgefässe bei Infarktpatienten hat die Angiographie den wesentlichen Nachteil, dass die MRT Scanner nicht schnell genug sind, um die Herzkranzgefässe abzubilden, die sich in ständiger Bewegung befinden und dass sich die Dynamik des Blutflusses nicht darstellen lässt. Letzteres ist vor allem auch wichtig bei der Untersuchung von Patienten mit Hirninfarkt oder anderen neurologischen Erkrankungen.

In einem inhomogenen Magnetfeld erfahren diamagnetische Stoffe (diamagnetisch sind die meisten in der Technik, Umwelt und Natur relevanten Stoffe) eine Kraft, die in Richtung Abnahme des Magnetfeldes gerichtet ist (siehe Gouy-Waage zur Messung der diamagnetischen Suszeptibilität). Bekannt ist auch, dass die Kraft, die auf diamagnetische Probe wirkt, nicht nur von der Stärke des Magnetfeldgradienten und der diamagnetischen Suszeptibilität der Probe abhängt, sondern auch von der magnetischen Suszeptibilität der Umgebung, in der sich die diamagnetische Probe befindet. Die Kraft ist proportional zur Differenz der magnetischen Suszeptibilität der Probe und der Umgebung. Wegen der Ähnlichkeit mit dem Auftrieb nennt man das Prinzip auch das magnetische Archimedes Prinzip.

Die Nutzung magnetischen Archimedes-Prinzips ist zur Zeit dadurch begrenzt, dass sich die Kräfte, die auf diamagnetische Objekte wirken, nur durch Änderung des äußeren Magnetfeldgradienten steuern lassen. Eine gezielte, selektive Bewegung von Einzelobjekten innerhalb des Magnetfeldes ist so nur bedingt möglich. Erreichen könnte man dies, indem man die magnetische Suszeptibilität des Mediums, in dem sich die Objekte befinden, mit hoher räumlicher Auflösung gezielt verändert. Das ist bislang nur in Festkörpern (und auch da nur bedingt) gelungen. In einem Festkörper kann allerdings naturgemäß keine freie Bewegung von Objekten stattfinden.

Aus der Dissertation "Ligandgetriebener lichtinduzierter Spin-Crossover in Einzelmolekülen bei Raumtemperatur" von C. Bornholdt (2008) sind lichtschaltbare Komplexe bekannt, welche bei 365 nm (UV-Bereich) vom low-Spin in den high-Spin-Zustand wechseln können. Der paramagnetische high-Spin-Zustand dieser Komplexe ist nur bei tiefen Temperaturen und im festen Zustand, nicht in Lösung, stabil.

Anwendungsgebiete photoschaltbarer Einzelmolekülmagnete sind insbesondere die Informationspeicherung, das große Potential dieser Verbindungen liegt in der hohen Speicherkapazität (S. Kawata, Y. Kawata, Chem. Rev. 2000, 100, 1777).

Die Eigenschaft den Spin-Crossover eines Komplexes der Übergangsmetalle durch Bestrahlen mit Licht herbeizuführen, nennt man LIESST (Light-Induced Excited Spin-State Trapping)-Effekt. Den LIESST-Effekt bei Raumtemperatur beobachtet man nur im Festkörper.

Bekannte SCO-Verbindungen (in der Regel auf Fe(II) oder Fe(III) Basis) lassen sich zwar durch Licht von low Spin nach high Spin schalten, aber im isolierten Molekül ist der paramagnetische high Spin Zustand nur bei sehr niedrigen Temperaturen (< 50K) langlebig. Der high Spin Zustand kehrt sehr schnell zum thermodynamisch stabileren (als Kontrastmittel inaktiven) low Spin Zustand zurück.

Es ist Aufgabe der Erfindung einen photosensitiven molekularen Schalter bereitzustellen, der sich unter Lichteinfluss von einem magnetischen Zustand in den anderen schalten lassen und auch im thermodynamisch instabileren (in der Regel paramagnetischen) Zustand bei Raumtemperatur stabil ist. Insbesondere ist es Aufgabe, einen als Kontrastmittel für die Magnetresonanztomographie geeigneten molekularen Schalter bereitzustellen.

Die Aufgabe wird durch die in den Ansprüchen beschriebenen Verbindungen gelöst. Die Unteransprüche geben besondere Ausgestaltungen der Erfindung wieder.

Mit der vorliegenden Erfindung werden Übergangsmetallkomplexe bereitgestellt, die sich mit Licht von einem magnetischen Zustand in den anderen schalten lassen und auch in Lösung im high spin und low spin Zustand bei Raumtemperatur stabil sind.

Die Erfindung schafft Übergangsmetallkomplexe, die sich mit Licht einer physiologisch gut vertretbaren Wellenlänge, d.h. im sichtbaren Bereich von einem magnetischen Zustand in den anderen schalten lassen und auch im paramagnetischen Zustand bei Raumtemperatur stabil sind.

Die Erfindung erlaubt es, die magnetische Suszeptibilität einer Lösung gezielt, mit hoher räumlicher Auflösung zu verändern und damit eine berührungslose, mit Licht gesteuerte Bewegung von Objekten in der Lösung in einem inhomogenen Magnetfeld zu bewirken.

Schließlich stellt die Erfindung einen photosensitiven molekularen Schalter zum Aufbau von Speichermedien bereit.

Es hat sich gezeigt, dass man erstmalig im paramagnetischen Zustand und in Lösung stabile LD-CISCO (light-driven coordination-induced spin crossover)-Komplexe erhält, wenn man einen schaltbaren Übergangsmetallkomplex bestehend aus Übergangsmetallion, Chelat-Ligand mit einem oder mehreren Substituenten, photochromen System, axialem Liganden und Henkel verwendet. Wobei das Übergangsmetallion ausgewählt ist aus der Gruppe $Mn^{2+}$, $Mn^{3+}$, $Fe^{2+}$, $Fe^{3+}$, $Co^{2+}$ und $Ni^{2+}$ und der Chelatligand derartig ausgebildet ist, dass er zur Ausbildung von Komplexen mit angenähert quadratisch planarer (e.g. für $Ni^2$) oder angenähert quadratisch pyramidaler (e.g. für $Fe^{2+}$) Konfiguration führt, und der oder die Substituenten so geartet sind, dass bereits bei der Änderung der Koordinationszahl um eins (z.B. von 4-facher zu 5-facher Koordination oder von 5-facher zu 6-facher Koordination) eine Änderung des Spinzustandes eintritt) und das photochrome System bei Bestrahlung mit Licht isomerisiert und der axiale Ligand elektronenreich ist und der Henkel zur kovalenten Anknüpfung des Schalters an den Komplex so beschaffen ist, dass der axiale Ligand in einer der beiden Konfigurationen des Schalters genau in der Mitte 1,8-2,4 Å über dem Übergangsmetallion zu liegen kommt und der Henkel zur kovalenten Anknüpfung so beschaffen ist, dass er einen Energietransfer des angeregten Zustandes vom molekularen Schalter auf den quadratisch planaren Komplex einschränkt bzw. nicht zulässt.

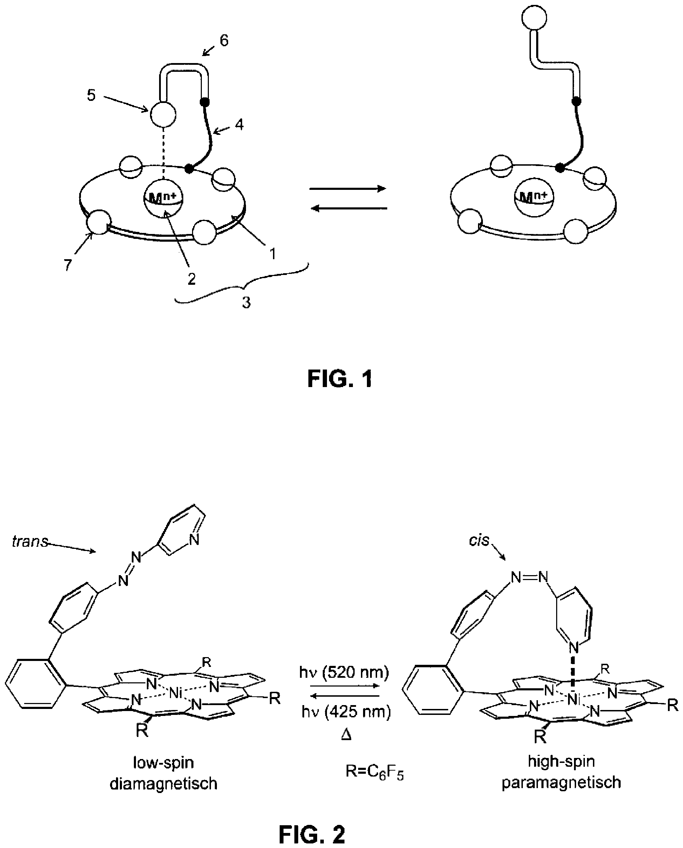

Eine schematische Darstellung des Prinzips findet sich in Fig. 1.

Grundsätzlich sind alle quadratisch planaren Nickel-, Kobalt-, Eisen-, oder Mangan-Komplexe geeignet, die einen schaltbaren Liganden besitzen, mit dem man reversibel eine der beiden axialen Koordinationsstellen besetzen und wieder entfernen kann und deren Spinzustand sich durch Assoziation bzw. Dissoziation des axialen Liganden gezielt ändern lässt.

Im Ni (II) gibt es in Abhängigkeit von der Koordinationssphäre zwei verschiedene Spinzustände. Bezeichnet man mit $n$ die Koordinationszahl, so zeigt $Ni^{2+}$ in quadratischplanaren Komplexen ($n$=4) den diamagnetischen low Spin -Zustand (S=0), in quadratisch bipyramidalen Komplexen ($n$=6) immer den paramagnetischen high-Spin-Zustand (S=1). Quadratisch pyramidale Komplexe ($n$=5) können je nach der Natur der verwendeten Liganden high Spin oder low Spin sein. Werden zu einem quadratisch-planarem Ni (II) - Komplex zwei axiale Liganden hinzugefügt und damit die Koordinationszahl von 4 auf 6 erhöht, so kommt es zu einem Wechsel des Spinzustandes von low-spin zum high-spin Zustand.

Als Chelat-Liganden im quadratisch planarem Komplex kommen Liganden in Frage, die eine quadratisch planare Konfiguration vorgeben, insbesondere Porphyrine, Phthalocyanine, Porphyrazine, Naphthocyanine, Chlorine, Bakteriochlorine, Corrine, Corrole und andere Tetrapyrrole und deren Heteroanaloga, sowie Salene, Glyoxime, Triethylentetramine, Cyclam- (1,4,8,11-Tetraazacyclotetradecan) und 1,4,8,11-Tetrathiocyclotetradecan-Derivate.

Als axiale Liganden kommen Liganden mit starken Elektronen-Donor-Eigenschaften in Frage insbesondere substituierte Pyridine, Imidazole, Pyrrolidine, Piperidine, Pyrrole, Pyrazine, Triazole Tetrazole, Alkylamine, Posphine, Arsine, Thiole, Thioether, und Tetrahydrothiophen.

Als photochrome Systeme eignen sich insbesondere solche Systeme die bei Bestrahlung mit Licht im Wellenlängenbereich zwischen 300 und 900 nm eine Isomerisierung erfahren, besonders bevorzugt sind Azobenzol, Phenylazopyridin oder Azopyridin, Thioindigo, Hemithioindigo, Spiropyrane, Spiroindolizine, Diarylethene und Fulgide.

Die kovalente Anknüpfung des molekularen Schalters an den quadratisch planaren Komplex wird über geeignete Henkel hergestellt. Dieses stellt einen besonderen Vorteil der Erfindung dar. Der axiale Ligand ist über kovalente, chemische Bindungen direkt an den Chelat-Liganden gebunden.

Im Prinzip kann man den Aufbau und die Funktion des schaltbaren Übergangsmetallkomplexes mit einem Plattenspieler vergleichen. Der Plattenteller entspricht dem quadratisch planaren Chelat-Komplex und der Tonarm wird vom Henkel (kovalente Anknüpfung) und dem photochromen System gebildet. Letzteres führt die lichtgesteuerte Bewegung des Tonarms aus. Die Nadel des Tonabnehmers ist das freie Elektronenpaar am axialen Liganden (siehe Fig. 1). Es hat sich gezeigt, dass durch geeignete Konstruktion zwei oder mehr der oben genannten Funktionen in einem Molekül vereint werden können. So enthält das Azopyridin als schaltbares Molekül im Beispielmolekül in Fig. 2 bereits die Nadel (freies Elektronenpaar am Pyridin-Stickstoff) und es vermittelt über den Phenylring gleichzeitig einen Teil der kovalenten Verknüpfung. Alle genannten molekularen Bauteile müssen geometrisch so aufeinander abgestimmt sein, dass beim lichtinduzierten Isomerisieren des schaltbaren Moleküls in einem der beiden Schaltzustände das freie Elektronenpaar des axialen Liganden senkrecht zum quadratisch planaren Komplex und in einem Abstand von etwa 1,8-2,4 Å zum Metallion (je nach axialem Ligand, Metallatom und Oxidationszustand) zu liegen kommt und eine axiale Koordination zum Metallion eingeht. Im anderen Schaltzustand muss das freie Elektronenpaar einen von 90° verschiedenen Winkel und eine größere Entfernung (>2,4 Å) besitzen und darf nicht mit dem Metallion koordinieren.

Im Fall von Porphyrin als quadratisch planaren Chelat-Liganden, gibt es grundsätzlich zwei Möglichkeiten zur kovalenten Anknüpfung des Henkels: (a) in *meso*-Position und (b) in *β*-Position. Im Fall des Phenylazopyridins als schaltbarem Liganden gibt es zunächst drei verschiedene Regioisomere mit der Azogruppe in 2-, 3- und 4-Position am Pyridin. Für das 3-Phenylazopyridin gibt es wiederum 7 Anknüpfungsmöglichkeiten an den Henkel: in 2-, 4-, 5- und 6-Position des Pyridinrings und in 2-, 3- und 4-Position am Phenylring. Geht man nun vom Porphyrin und Azopyridin als Grundbausteinen aus, ergibt sich eine Vielzahl von Kombinationsmöglichkeiten. Zu jeder muss nun ein geeigneter Henkel gefunden werden, der Porphyrin und Azobenzol so verbindet, dass ein funktionsfähiges System entsteht. Dazu muss der Henkel im Wesentlichen zwei Voraussetzungen erfüllen: (a) er muss geometrisch so beschaffen sein, dass er die axiale Koordination bzw. Dekoordination bewirkt, (b) er muss elektronisch so abgestimmt sein, dass keine direkte, durchgehende Konjugation des schaltbaren Moleküls mit dem quadratisch planaren Metallkomplex stattfindet, da sonst das Schaltverhalten beeinflusst wird. (c) er sollte möglichst wenige Freiheitsgrade (Drehung um Einfachbindungen) besitzen um ein Wegdiffundieren des axialen Liganden zu verhindern. Um beispielsweise ein Nickel-Porphyrin in *meso*-Position mit einem 3-Phenylazopyridin in 2-Position (*ortho* zur Azogruppe) am Phenylring zu verbinden, muss der Henkel drei CH$_2$-Gruppen besitzen, wobei die CH$_2$-Gruppen in erster Näherung, z.B. durch O, oder NH ersetzt werden können, um die Synthetisierbarkeit zu erleichtern. In dieser Geometrie koordiniert das Pyridin mit dem Nickel, wenn sich die Azo-Gruppe in *cis*-Stellung befindet und koordiniert nicht, wenn die Azo-Gruppe in *trans*-Stellung vorliegt. Um ein Nickel-2,3-Dihydro-porphyrin in 2-Position mit einem 3-Phenylazopyridin in 2-Position (*ortho* zur Azogruppe) am Phenylring zu verbinden ist idealerweise eine Kette von drei CH$_2$-Gruppen notwendig. Auch hier gilt, dass eine oder mehrere CH$_2$-Gruppen durch Atome oder Gruppen ähnlicher Geometrie ersetzt sein können. Mit einem solchen Henkel würde der Pyridin-Stickstoff koordinieren, wenn die Azo-Gruppe in *trans*-Konfiguration vorliegt und nicht *cis*-Konfiguration.

In Fig. 2 ist exemplarisch ein erfindungsgemäßer Azopyridin funktionalisierten Ni-Prophyrin-Komplex zu sehen, der hergestellt und dessen Schaltfähigkeit nach den obigen Prinzipien experimentell bewiesen wurde.

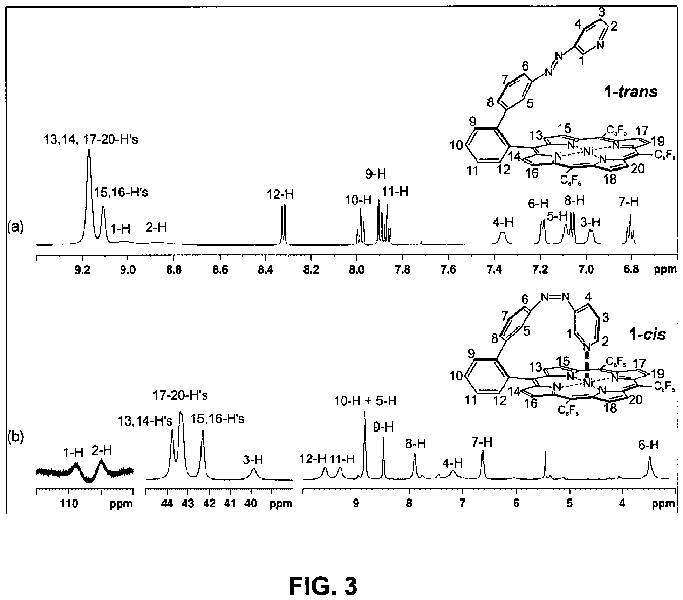

Fig. 3 zeigt die NMR-Spektren der beiden Isomere eines erfindungsgemäßen Azopyridin funktionalisierten Ni-Prophyrin-Komplexes. Der Übergang vom *trans*- in den *cis*-Zustand ist von einer starken Tieffeldverschiebung und Verbreiterung der Signale begleitet.

Aus Fig. 1 ist das Prinzip der erfindungsgemäßen schaltbaren Komplexe ("Plattenspieler"-Komplexe) zu erkennen. Durch Bestrahlung mit Licht der Wellenlänge zwischen 500 und 520 nm oder 330 nm oder 380 nm, bevorzugt 510 nm geht das Molekül an der Diazo-Funktionalität (-N=N-) vom *trans* ((*E*)-Isomer) zum *cis* ((*Z*)-Isomer) über. In der *cis*-Konfiguration besetzt der Stickstoff des Pyridins mit seinem freien Elektronenpaar die axiale Koordinationsstelle. Es kommt so zu einem Übergang vom diamagnetischen low Spin-Zustand in der *trans*-Konfiguration zu einem paramagnetischem high Spin-Zustand. Die Bestrahlung mit Licht der Wellenlänge 420 - 450 nm bevorzugt 425 nm führt zu einem erneuten Wechsel der Konfiguration an der Azo-Funktionalität. Das Molekül geht wieder in die *cis*-Konfiguration über, damit kommt es zu einer Änderung des Spinzustandes vom paramagnetischen high Spin zum diamagnetischen low Spin-Zustand.

Ein besonderer Vorteil der erfindungsgemäßen Komplexe liegt darin, dass hier zum Schalten von einem Zustand in den anderen, Licht aus dem Frequenzbereich des sichtbaren Lichtes verwendet wird. Diese Wellenlängen sind physiologisch weit besser verträglich als zum Beispiel Licht aus dem UV-Bereich, damit ist es möglich derartige Komplexe auch im Bereich des menschlichen Körpers einzusetzen.

Wegen der extrem hohen Extinktion der *cis*-Verbindung (epsilon= 230 000 1 mol$^{-1}$ cm$^{-1}$, bei 425 nm) und der hohen Quantenausbeute (~15%) ist die erfindungsgemäße Schaltung der erfindungsgemäßen Komplexe in diesem Wellenlängenbereich sehr empfindlich, so dass keine großen Intensitäten und Energien benötigt werden. Damit sind insbesondere die wichtigsten physikalischen Voraussetzungen für eine Anwendung beispielsweise als lichtschaltbare MRI-Kontrastmittel gegeben.

Die Schaltvorgänge bei den erfindungsgemäßen Verbindungen haben sich als extrem reversibel erwiesen. Auch nach mehr als 10.000 Schaltzyklen zeigen sich keine Nebenreaktionen oder Ermüdungserscheinungen. Dieses Ergebnis zeigt sich auch bei Raumtemperatur und unter Zutritt von Luft. Die Experimente mit den 10.000 Schaltzyklen wurden bei 20 °C und Zutritt von Luft durchgeführt.

Die Lebensdauer des paramagnetischen Zustands der erfindungsgemäßen Komplexe ist sehr hoch. Die Halbwertzeit des paramagnetischen Zustandes bei Raumtemperatur (21 °C) beträgt mehrere Monate. Die Bestimmung der Halbwertszeit erfolgte dabei sowohl durch NMR-, als auch durch UV-Spektroskopie.

Ein weiterer Vorteil der erfindungsgemäßen molekularen Schalter liegt darin, dass sie auch in Lösung stabil sind und schalten.

Dass bedeutet unter anderem, dass sie direkt als schaltbare Kontrastmittel in die Blutbahn gespritzt werden können, Die Nachteile von Kontrastmitteln, die nur als Festkörper aktiv sind, werden so vermieden. Diese Nachteile sind zum einen die notwendige Verkapselung der Festkörper zum Schutz gegen die Auflösung zum Anderen können die nur als Suspension einbringbaren Partikel die Blutgefäße verstopfen.

Ein besonderer Vorteil der erfindungsgemäßen molekularen Schalter liegt daher darin, dass hier ein Einsatz als schaltbare Kontrastmittel in der Magnetresonanztomographie möglich ist. Nur im paramagnetischen Zustand sind die erfindungsgemäßen Komplexe als Kontrastmittel wirksam. Schaltbare Kontrastmittel haben daher den Vorteil, dass man das Signal-RauschVerhältnis durch Schalten verbessern kann, wenn man abwechselnd Bilder mit ein- und ausgeschaltetem Kontrastmittel aufnimmt und dadurch den Hintergrund wegmitteln kann.

Mit einem schaltbaren Kontrastmittel kann auch der Blutfluss verfolgt werden, was mit einem herkömmlichen Kontrastmittel nicht möglich ist. Man belichtet an einem bestimmten Punkt in einem Blutgefäß, schaltet das Kontrastmittel ein und verfolgt in mehreren, hintereinander folgenden Aufnahmen die Ausbreitung. Mit einem schaltbaren Kontrastmittel kann man so auch die Schaltfrequenz des Kontrastmittels auf die Herzfrequenz abgleichen und Bilder der gleichen Bewegungsphase sammeln und zu einem statischen Bild zusammenführen.

Eine sehr vorteilhafte Anwendung der erfindungsgemäßen - im paramagnetischen Zustand und auch in Lösung stabilen - molekularen Schalter ist die berührungslose Bewegung von Objekten, welche im Folgenden näher erläutert wird.

Bereits in den 60er Jahren hat man das Magnethydrostatische Prinzip (auch als magnetisches Archimedes Prinzip genannt) vorgeschlagen um Erze zu trennen (U. Andres. Magnetohydrodynamic and Magnetohydrostatic Methods of Mineral Separation; Wiley: New York, 1976). Das gleiche Prinzip ist vor kurzem vorgeschlagen worden um die Dichte von diamagnetischen Stoffen zu bestimmen (K. A. Mirica, S. S. Shevkoplyas, S.T. Phillips, M. Gupta, G. M. Whitesides, J. Am. Chem. Soc. 2009, 131, 10049-10058). Man benötigt dazu starke Magneten, mit dem man ein inhomogenes Magnetfeld erzeugt, dessen Gradient möglichst parallel zur Erdbeschleunigung liegt (d.h. senkrecht zur Erdoberfläche) und dessen Vektor der Erdanziehung parallel gerichtet ist (d.h. die Stärke des Magnetfeldes B nimmt nach oben ab). In dieses inhomogene Magnetfeld bringt man eine paramagnetische Flüssigkeit ein. Dabei kann es sich um eine Lösung eines paramagnetischen Salzes handeln (z.B. $MnCl_2$, $FeCl_2$, $CoBr_2$, $NiSO_4$, $Dy_2(SO_4)_3$, oder um eine Suspension eines ferromagnetischen Materials (z.B. Magnetit ($Fe_3O_4$) oder künstliche Ferrite (z.B. $Fe_3Y_5O_{12}$)). In der Flüssigkeit befinden sich die diamagnetischen Proben. Auf die Proben wirken nun zwei verschiedene Kräfte ein. 1. Die Gravitationskraft, vermindert um den Auftrieb in der Flüssigkeit und 2. eine magnetische Kraft, die in Richtung Abnahme des Magnetfeldes (d.h. der Erdanziehung entgegen) gerichtet ist. Die Graviationskraft $F_{grav}$ ist an allen Stellen gleich und nur vom Dichteunterschied der Flüssigkeit und der Probe abhängig.

$$F_{grav} = (\rho_p - \rho_m)Vg$$

$\rho_p$: Dichte der Probe
$\rho_m$: Dichte des Mediums
V: Volumen der Probe
g : Gravitationskonstante

Die magnetische Kraft hängt von der Stärke des Magnetfeldes, des Magnetfeldgradienten und der Differenz der magnetischen Suszeptibilität der Probe und der Lösung ab.

$$F_{mag} = [(\chi_p-\chi_m)/\mu_0]\ V\ B_z\ dB_z/dz$$

$\chi_p$: magnetische Suszeptibilität der Probe
$\chi_m$: magnetische Suszeptibilität des Mediums
$\mu_0$: magnetische Permeabilität des Vakuums
$B_z$: magnetische Induktion in z-Richtung (senkrecht zur Erdoberfläche)
$dB_z/dz$: Magnetfeldgradient in z-Richung

Die magnetische Kraft verringert sich mit zunehmendem Abstand vom Magneten. Wenn Gravitationskraft und magnetische Kraft einander entgegengesetzt sind, gibt es eine bestimmte Entfernung vom Magneten, bei der sich eine Probe im Gleichgewicht befindet, da sich an dieser Stelle beide Kräfte aufheben. Die Probe schwebt dann bewegungslos an einem Ort, der bei konstanten äußeren Parametern nur noch von der Dichte und der magnetischen Suszeptibilität der Probe abhängt.

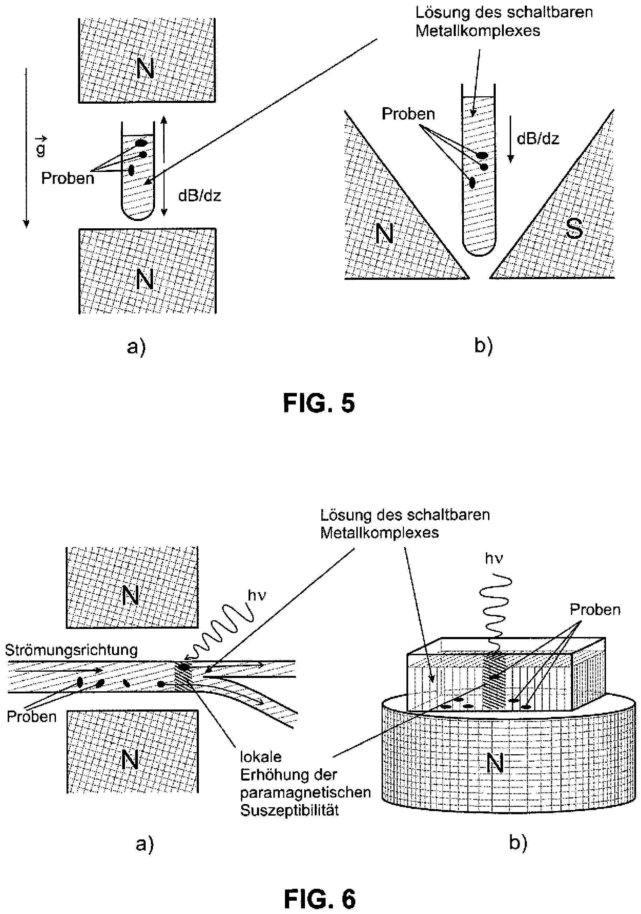

Der praktische Aufbau einer solchen Anlage ist einfach (siehe Fig. 5). Den MagnetfeldGradienten erzeugt man z.B. dadurch, dass man entweder zwei Magneten im Sinne einer sogenannten *anti*-Helmholtz-Anordnung aufstellt (gleichnamige Pole stehen einander vertikal gegenüber so, dass sie sich abstoßen, z.B. Nordpol gegenüber Nordpol). Dies führt zu einem weitgehend konstanten Feldgradienten (die magnetische Induktion B fällt von jedem Magneten zur Mitte hin linear ab). Die vertikale Komponente von B (in z-Richtung) ist genau zwischen den Magneten Null. Oder man bringt die beiden Magneten in einer Anordnung zusammen, bei der Nordpol und Südpol einander waagerecht gegenüberstehen. Der Abstand der beiden Pole muss eine keilförmige Form besitzen (unten näher zusammen als oben) (Fig. 5). Im einfachsten Fall reicht ein einzelner Magnet, dessen Nord- oder Südpol nach oben steht (Fig. 6b). Das inhomogene Magnetfeld kann entweder durch starke Permanentmagneten (z.B. $Nd_2Fe_{14}B$ oder $SmCo_5$ oder $Sm_2Co_{17}$) oder Elektromagneten erzeugt werden.

Neuartig an unserer Erfindung ist nun, dass man statt der Lösung des paramagnetischen Salzes oder der Suspension paramagnetischer Partikel, die Lösung des erfindungsgemäßen molekularen Schalters in das homogene Magnetfeld einbringt. Im Gegensatz zur einfachen Metallsalzlösung kann man nun die magnetische Suszeptibilität der Lösung mit Licht (z.B. durch Laser mit zwei verschiedenen Wellängen) mit hoher räumlicher Auflösung reversibel ändern. Ändert man die magnetische Suszeptibilität in der Umgebung einer bestimmten Probe durch Einstrahlen von Licht, ändert sich die auf die Probe einwirkende magnetische Kraft. Die Probe wird angehoben, wenn man die paramagnetische Suszeptibiliät erhöht (Schalten in den high spin Zustand) oder abgesenkt (Schalten in den low spin Zustand). Zahlreiche praktische Anwendungen sind denkbar. In einem Flüssigkeitsstrom den man z.B. in einem Rohr durch das Magnetfeld führt und der kleine Objekte, wie z.B. Bakterien oder Zellen enthält kann man nun durch gezieltes Einstrahlen von Licht einzelne Objekte aussortieren, indem man sie anhebt oder absenkt und entsprechend in eine Abzweigung lenkt (Fig. 6 a)). Auch das Platzieren von Objekten z.B. für mikroskopische Untersuchungen oder das genaue Positionieren von Bauteilen in der Mikroelektronik kann durch die berührungslose Bewegung mit Licht erfolgen. Dazu wird ein Gefäß mit der Lösung des erfindungsgemäßen, schaltbaren Metallkomplexes auf die Oberfläche des Pols eines Magneten gestellt. Die Stärke des Magnetfeldes, das spezifische Gewicht der Probe und der Lösung sind so einzustellen, dass die magnetische Levitation nicht ausreicht, um die Probe zum Schweben zu bringen ($F_{grav} > - F_{magn}$), wenn sich der Metallkomplex im low spin Zustand befindet. Durch Einstrahlen von Licht in der Umgebung einer Probe und Isomerisieren des Komplexes in den high spin Zustand wird die Probe vom Boden des Gefäßes angehoben, durch Anlegen eines horizontalen Magnetfeldgradienten seitwärts bewegt und durch Zurückschalten des Metallkomplexes in den low spin Zustand wieder abgesenkt (Fig. 6 b)).

Eine weitere Anwendung der erfindungsgemäßen molekularen Schalter liegt im Bereich der optischen Speichermedien. Das Schreiben und Löschen der Information erfolgt dabei durch das Schalten der molekularen Schalter nach Bestrahlung mit Licht der geeigneten Wellenlänge. Im Falle der in Fig. 2 gezeigten Azopyridin funktionalisierten Ni-Prophyrin-Komplexen ist das die Wellenlänge von 520 nm bzw. 425 nm. Für das Auslesen der Information wird Licht einer Wellenlänge verwendet, welche nicht zur Isomerisierung, dass heißt dem Schalten führt, aber von nur einem der beiden Isomere (cis oder trans) absorbiert wird. Im Falle der in Fig. 2 gezeigten Azopyridin funktionalisierten Ni-Prophyrin-Komplexen ist das z.B. eine Wellenlänge von 544 nm.

Die Herstellung der erfindungsgemäßen molekularen Schalter erfolgt nach einem mehrstufigen Verfahren.

1. Das photochrome System wird im einfachsten Fall aus den Komponenten 3-Bromnitrobenzol und 3-Aminopyridin hergestellt. In Frage kommen auch in 2-, 4-, 5-, oder 6-Position substituierte 3-Bromnitrobenzole. Statt der Bromverbindung kommen auch die entsprechenden Chlor- und Iodverbindungen in Frage oder andere Substituenten, die sich für die später durchgeführte Suzukikupplung eignen (z.B. Triflat). Ebenfalls in Frage kommen auch substituierte Aminopyridine. Vorteilhaft sind hier elketronenliefernde Substituenten in 4-Position. Zur Kupplung der Komponenten wird zunächst 3-Bromnitrobenzol (oder ein entsprechendes Derivat) mit Zn zum entsprechenden Hydroxylamin reduziert und mit $FeCl_3$ zur Nitrosoverbindung oxidiert, die nicht isoliert sondern direkt mit 3-Aminopyridin (oder einem entsprechenden Derivat) zur entsprechenden Azoverbindung, dem 3-((3-Bromphenyl)diazenyl)pyridin (oder zum entsprechenden Derivat) kondensiert wird.

2. Im nächsten Schritt wird der Henkel, der im vorliegenden Fall nur aus einem metasubstituierten Phenylring besteht, an der Azoverbindung angebracht. Dazu wird die Brom-substituierte Azoverbindung (oder das entsprechende Derivat) im Sinne einer Suzuki-Kupplung mit (2-Formylphenyl)boronsäure umgesetzt. Letztere Verbindung kann auch in 3-, 4-, 5- oder 6-Stellung substituiert sein, wobei elektronenziehende Substituenten vorteilhaft sind. Das Produkt 2-(Pyridin-3-ylazo)benzaldehyd oder das entsprechende Derivat wird als Komponente für die nachfolgende Cyclisierung zum Aufbau des Porphyrinringes verwendet.

3. Zur Synthese des Porphyrinringes benötigt man drei Komponenten im Verhältnis 4:3:1:

   a) Pyrrol oder ein in 3- oder 4-Stellung substituiertes Pyrrol.
   b) Pentafluorbenzaldehyd (A) oder ein anderes, vorzugsweise elektronenziehende Substituenten tragendes Benzaldehyd.
   c) 2-(Pyridin-3-ylazo)benzaldehyd (B) (4), welches in 2-, 4-, 5- oder 6-Stellung am Pyridin substituiert sein kann, vorzugsweise mit einem elektronenliefernden Substituenten in 4-Position (z.B. 2-((4-Methoxypyridin-3-yl)diazenyl)benzaldehyd). In Frage kommen auch 2-(Pyridin-3-ylazo)benzaldehyde, die am Phenylring in 2-, 3-, 4- und 5-Position relativ zur Azo-Gruppe substituiert sind. Der Formyl-tragende Ring kann ebenfalls substituiert sein.

Die Komponenten kondensieren Säure- oder Lewissäure-katalysiert (mixed-Aldehyd-Variante der Adler-Methode) zu einem statistischen Gemisch von meso-substituierten Porphyrinen (Substitutionsmuster: $A_4$, $A_3B$, cis $A_2B_2$, trans-$A_2B_2$, $AB_3$, $B_4$) wobei, gleiche Reaktivität der beiden Aldehyde vorausgesetzt, das gewünschte $A_3B$ Porphyrin als Hauptprodukt entsteht. Die Ausbeute am gewünschten Produkt lässt sich erhöhen, wenn man Pentafluorbenzaldehyd mit Pyrrol zum Dipyrromethan 2 vorkondensiert und isoliert. Setzt man zwei Äquivalente Dipyrromethan 2 als Baustein mit jeweils einem Äquivalent Pentafluorbenzaldehyd und 2-(Pyridin-3-ylazo)benzaldehyd um, können sich nur noch drei Produkte bilden (A₄, A₃B, trans-A₂B₂). Die Ausbeute am gewünschten Produkt ist entsprechend höher und die Trennung des Produktgemisches durch Chromatographie vereinfacht sich.

4. Zunächst entsteht bei der Cyclisierung ein Porphyrinogen, welches durch Luft oder vorteilhafter mit DDQ oder Chloranil zum Porphyrin oxidiert wird.

5. Durch Reaktion mit einem Ni(II)-Salz, z.B. Nickelacetonylacetat wird das Nickel-Porphyrin gebildet, welches die erfindungsgemäße Verbindung darstellt.

Alternativ kann die Reihenfolge der C-C Verknüpfungen in der Synthese vertauscht werden. So kann man zunächst den Aufbau des Porphyrin-Ringes mit zwei Äquivalenten Dipyrromethan und je einem Äquivalent Pentafluorobenzaldehyd und 2-Brombenzaldehyd oder einem anderen für die spätere Suzukikupplung geeigneten, in 2-Position substituierten Benzaldehyd durchführen. Am entstandenen mit drei Pentafluorphenyl- und einer 2-Bromphenyl-Gruppe substituierten Porphyrin wird dann das Bromatom gegen Boronsäure ausgetauscht und im Sinne einer Suzuki-Kupplung mit 3-((3-Bromphenyl)diazenyl)pyridin umgesetzt oder umgekehrt kann auch das Brom-substituierte Porphyrin mit (3-(Pyridin-3-yldiazenyl)phenyl)boronsäure gekuppelt werden. Auch kann die Einführung des Nickel-Ions in den Porphyrin-Ring nach dem Ringschluss statt im letzten Schritt erfolgen.

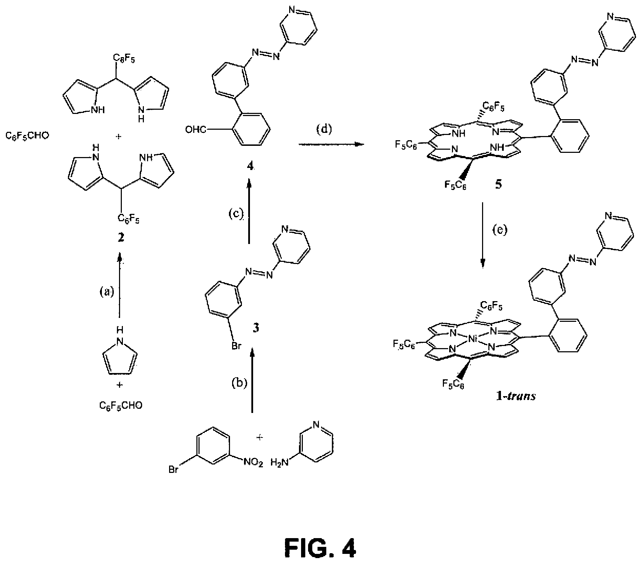

Exemplarisch die Allgemeinheit der Lehre nicht einschränkend wird hier die Synthese des erfindungsgemäßen Komplexes beschrieben. In Fig. 4 sind die zugehörigen Formeln dargestellt.

**b) Synthese von 3:**

Zu einer gerührten Lösung von 3-Bromnitrobenzol (8.08 g, 40 mmol) in Ethanol (150 ml), wird unter Stickstoffatmosphäre eine Lösung von NH₄Cl (3.21 g, 60 mmol) in 15 ml Wasser hinzugegeben. Die resultierende Mischung wird auf 40 °C erwärmt und eine klare Lösung erhalten. Zu dieser Lösung wird bei Raumtemperatur über einen Zeitraum von 20 Min langsam Zn Pulver (6.54 g, 100 mmol) zugegeben und anschließend weitere 2 h gerührt. Die Reaktionsmischung wird filtriert und mit Ethanol und dann mit Wasser gewaschen. Das vereinigte, leicht gelbliche Filtrat wird dann tropfenweise, unter Rühren zu einer eiskalten Lösung von FeCl₃.6H₂O (18 g, 66 mmol) in 200 ml H₂O gegeben. Ein grün-gelber Niederschlag bildet sich, der durch Filtration abgetrennt und mit Wasser gewaschen wird. Das Produkt wird zwei Tage lang an der Luft getrocknet und dieses Rohprodukt (7.2 g, 38.70 mmol) wird langsam bei 80 °C und Stickstoffatmosphäre zu einer gerührten zweiphasigen Lösung von 3-Aminopyridine (3.57 g, 38 mmol) in Toluol (30 ml) und 40% NaOH Lösung (20 ml) gegeben. Die Mischung wird 2.5 h unter Rückfluss gekocht. Dann lässt man die Reaktionsmischung auf Raumtemperatur erwärmen und extrahiert mit Ethylacetat (150 ml). Der Ethylacetat Extrakt wird mit Wasser (2 x 100 ml) gewaschen, über wasserfreiem Na₂SO₄ getrocknet und unter reduziertem Druck vom Lösungsmittel befreit. Das Rohprodukt wird durch Flash Chromatography auf Silicagel mit Ethylacetat und Cyclohexane (3:7) als Eluent gereinigt und man erhält 3 als einen reinen orange-roten Feststoff (3.47 g, 13.24 mmol, 35 %), $R_f$ = 0.53 (1:1 Ethylacetat und Cyclohexan).

Schmp.; 71-72 °C; IR(cm¹) : 3057 (w), 1570 (s), 1449 (m), 1422 (s), 812 (vs), 780 (vs), 696 (vs), 674 (vs), 616 (s), 547 (s), 533 (s), 480 (m); UV-vis (CHCl₃) ($\lambda_{max}$ in nm, Abs. units): 319 (0.66), 242 (0.41);
¹H NMR (CDCl₃, 500 MHz): $\delta$ = 7.42 (t, $J$ = 8.0 Hz, 1H, H-8), 7.45 (ddd, $J$ = 8.0, 4.7, 0.7 Hz, 1H, H-2), 7.63 (ddd, $J$ = 7.9, 1.95, 1.0 Hz, H-7), 7.91 (ddd, $J$ = 8.0, 1.85, 1.0 Hz, H-9), 8.08 (t, $J$ = 1.85 Hz, 1H, H-11), 8.13 (ddd, $J$ = 8.20, 2.34, 1.6 Hz, 1H, H-3), 8.72 (dd, $J$ = 4.72, 1.6 Hz, 1H, H-1), 9.20 (dd, $J$ = 2.4, 0.5 Hz, 1H, H-5); ¹³C NMR (CDCl₃, 125 MHz) $\delta$ = 123.14 (C-9), 123.24 (C-10), 124.03 (C-2), 124.87 (C-11), 126.97 (C-3), 130.57(C-8), 134.35 (C-7), 147.55 (C-4), 147.61 (C-5), 152.24 (C-1), 153.31 (C-6) ppm. MS [m/z (%)] 262.0 (100), 261.0 (49), 260.0 (97), 259.0 (32); Elementaranalyse: Ber. für C₁₁H₈BrN₃: C 50.41; H 3.08; N 16.03; gef.: C 50.50; H 3.07; N 16.01.

**c) Synthese von 4:**

Zu einer gerührten Lösung von 3 (1.00g, 3.81 mmol) in trockenem Toluol (30 ml) werden unter Stickstoff nacheinander 2-Formylphenyl-boronsäure (630 mg, 4.2 mmol), Ethanol (10 ml), K₂CO₃ (2 ml, 2 M Lösung in Wasser) and Pd(PPh₃)₄ (60 mg) gegeben. Dann wird die Lösung heftig gerührt und auf 90 °C erhitzt. Der Verlauf der Reaktion wird durch Dünnschichtchromatographie verfolgt. Nach 16 stündigem Erhitzen (Ölbad) wird die Mischung auf Raumtemperatur abgekühlt und mit Ethylacetat (100 ml) extrahiert. Die organische Schicht wird abgetrennt und mit Wasser (2 x 50 ml) gewaschen, über wasserfreiem Na₂SO₄ getrocknet und im Vakuum aufkonzentriert. Der dunkelorange-farbene Rückstand wird durch Flashchromatographie auf Silicagel mit Ethyl acetate und Hexane (3:7) als Eluenten gereinigt. Man erhält die Verbindung 4 als orange-roten Feststoff (1.04 g, 3.61 mmol, 95%), $R_f$ = 0.438 (40% Ehylacetat und Cyclohexan).

Schmp. 97-98 °C: IR (cm⁻¹): 3045 (w), 2862 (w), 1685 (s), 1589 (s), 1190 (s), 815 (vs), 758 (vs), 698 (vs), 647 (s), 614 (s), 510 (s); UV-vis (CHCl₃) ($\lambda_{max}$ in nm, abs. units): 320 (0.83), 257 (0.62), 242 (0.67);
¹H NMR (CDCl₃, 500 MHz): $\delta$ = 7.47 (dd, $J$ = 8.0, 4.7 Hz, 1H, H-2), 7.52-7.58 (m, 3H, H-9, H-15 and H-17), 7.66 (t, $J$ = 8.0 Hz, 1H, H-8), 7.69 (td, $J$ = 7.50, 1.4 Hz, 1H, H-16), 7.98 (t, $J$ = 2.0 Hz, 1H, H-11), 8.04 (dt, $J$ = 8.0, 1.6 Hz, 1H, H-7), 8.07 (dd, $J$ = 8.0, 1.5 Hz, 1H, H-14), 8.17 (dt, $J$ = 8.0, 2.0 Hz, 1H, H-3), 8.73 (dd, $J$ = 5.0, 1.5 Hz, 1H, H-1), 9.22 (d, $J$ = 2.30 Hz, 1H, C-5), 10.06 (d, $J$ = 0.6 Hz, 1H, H-18); $^{13}$C NMR (CDCl$_3$, 125 MHz) $\delta$ = 123.09 (C-7), 124.04 (C-2), 124.07 (C-11), 127.01 (C-3), 127.95 (C-14), 128.31 (C-9), 129.31 (C-8), 130.82 (C-15), 133.10 (C-17), 133.73 (C-13), 133.78 (C-16), 139.02 (C-10), 144.80 (C-12), 147.46 (C-5), 157.74 (C-4), 152.00 (C-1), 152.33 (C-6), 191.45 (C-18) ppm.
MS [m/z (%)] 277.1 (75), 278.1 (42), 287.1 (100); Elementaranalyse: ber. für C$_{18}$H$_{13}$N$_3$O: C 75.25; H 4.56; N 14.63; gef.: C 75.24; H 4.75; N 14.79.

**d) Synthese des metallfreien Porphyrins 5:**

In einem 500 ml Zweihals-Rundkolben werden CH$_2$Cl$_2$ (425 ml), Dipyrromethan **2** (2,15 g, 6,8 mmol), Aldehyd **4** (0,97 g, 3,3 mmol) und Pentafluorbenzaldehyd (0,66 g, 3,3 mmol) nacheinander zugegeben und der Kolben mit Aluminiumfolie umwickelt. Die Mischung wird heftig gerührt und bei Raumtemperatur 15 Min lang mit Stickstoff gespült. Dann wird BF$_3$.OEt$_2$ (0,98 g, 6,9 mmol) mithilfe einer Spritze zugegeben. Die Reaktionsmischung wird weitere 14 h lang bei Raumtemperatur gerührt Dann wird p-Chloranil (1,74 g, 7,1 mmol) in einer Portion zugegeben und die Mischung 7 h lang unter Rückfluss gehalten. Die Reaktionsmischung wird auf Raumtemperatur abgekühlt und mit Et$_3$N (1 ml, 7,2 mmol)) versetzt und weitere 30 Min lang gerührt. Schließlich wird das Lösungsmittel bis zur Trockene entfernt. Das dunkelgrüne Reaktionsprodukt wird durch wiederholte Flashchromatographie auf Silicagel mit CH$_2$Cl$_2$ als Eluent gereinigt und schließlich das gewünschte Produkt **5** als purpurfarbener Feststoff erhalten (152 mg, 0,143 mmol, 4,2 %). R$_f$ = 0,46 (2,5 % Etylacetat und CH$_2$Cl$_2$).
IR (rein, cm$^{-1}$, Intensität): 3318 (vw), 1723 (m), 1516 (s), 1494 (vs), 1261 (m), 1041 (m), 985 (vs), 917 (vs), 802 (s), 756 (vs), 701 (s);
UV-vis (CHCl$_3$) ($\lambda_{max}$ in nm, Abs. Einheiten): 586 (0.02), 512 (0.06), 418 (0.61), 347 (0.10); $^1$H NMR (CDCl$_3$, TMS, 500 MHz): $\delta$ = 2.92 (s, 2H, -NH), 6.50-6.53 (t, $J$ = 7.9 Hz, 1H, 7-H), 7.00-7.02 (dm, $J$ = 8.0 and 1.1 Hz, 1H, 8-H), 7.06-7.08 (dm, $J$ = 8.0 and 1.1 Hz, 1H, 6-H), 7.19-7.22 (dd, $J$ = 5.0, 3.4 Hz, 1H, 3-H), 7.47-7.49 (dm, $J$ = 8.1 and 2.1 Hz, 1H, 4-H), 7.75 (t, 1H, $J$ = 1.9 Hz, 5-H), 7.76-7.80 (m, 1H, 11-H), 7.93-7.96 (m, 2H, 10-H and 9-H), 8.18-8.20 (dm, $J$ = 7.5 and 1.1 Hz, 1H, 12-H), 8.51-8.52 (dd, $J$ = 5.0 and 1.7 Hz, 1H, 2-H), 8.71-8.75 (m, 3H, 1-H, 13-H and 14-H), 8.81-8.86 (m, 4H, 17-H, 18-H, 19-H and 20-H), 8.94-8.97 (d, $J$ = 4.8 Hz, 2H, 15-H, 16-H) ppm; $^{13}$C NMR (CDCl$_3$, 125 MHz) $\delta$ = 151.48 (C-5a), 150.53 (C-2), 147.41 (C-4a), 147.41 and 145.52 (6C, ortho F-C of C$_6$F$_5$ (X+Y+Z), 145.86 (C-1), 143.84 (C-9a), 143.21 and 141.13 (3C, para F-C of C$_6$F$_5$ (X+Y+Z)), 142.15 (C-8a), 139.27 (C-12a), 138.55 and 136.54 (6C, meta F-C of C$_6$F$_5$ (X+Y+Z)), 135.72 (C-12), 132.55 (C-8), 135.00-130.00 (br, 13-20, 13a-20a), 129.67 (C-10), 129.44 (C-9), 128.38 (C-7), 127.47 (C-4), 126.26 (C-11), 123.89 (C-5), 123.86 (C-3), 121.73 (C-A), 121.31 (C-6), 116.00-115.00 (br, 3C, q-C of C$_6$F$_5$ (X, Y, Z)), 102.94 (C-B and C-D), 102.00 (C-C) ppm;
$^{19}$F NMR (CDCl$_3$, 470 MHz): $\delta$ = -136.57 to -136.69 (m, 6- o-F of X, Y and Z), -151.63, -151.67, -151.72 and -151.76 (q, $J$ = 21 Hz, 3-p-F of X, Y and Z), -161.48 - -161.68 (m, 4- m-F of X, Y and Z);
MS [EI, m/z] 1065.0 (100), 1045.7 (8%), 959.0 (10%), 532.8 (20%).

**e) Synthese des Ni-Porphyrin (1):**

Zu einer gerührten Lösung von Porphyrin **5** (65 mg, 0.0610 mmol) in Toluol (20 ml) werden Ni(acac)$_2$ (230 mg, 0.785 mmol) unter Stickstoffatmosphäre hinzugegeben. Die Mischung wird 5 Tage lang refluxiert und der Fortgang der Reaktion wird durch Dünnschichtchromatographie verfolgt. Dann wird die Reaktionsmischung auf Raumtemperatur abgekühlt und das Lösungsmittel im Vakuum bis zur Trockene entfernt. Das Rohprodukt wird durch Flashchromatographie auf Silicagel mit 1 % Ethylacetat in CH$_2$Cl$_2$ als Eluent gereinigt und man erhält ein purpurfarbenes Produkt (rote Lösung) (50 mg, 0,0446 mmol, 73 %); R$_f$ = 0.46 (2,5 % EtOAc and CH$_2$Cl$_2$). Beim Stehenlassen im Licht erscheinen beim Trennen auf einer Dünnschichtchromatographieplatte zwei Spots die man **1-cis** und **1-trans** zuordnen kann (Eluent: 10 % Ethylacetat in CH$_2$Cl$_2$).
IR (rein, cm$^{-1}$, Intensität): 1517 (s), 1485 (vs), 1345 (m), 1059 (m), 985 (vs), 957 (vs), 937 (s), 800 (m), 761 (vs), 701 (s); UV-vis (in CH$_3$CN, 20 °C) $\lambda_{max}$ [nm], ($\in$/10$^3$ [L.mol$^{-1}$·cm$^{-1}$]): **1-trans** 323 (28.1), 406 (170.6), 524 (12.9), 557 (8.7); **1-cis** 321 (15.5), 421 (228.9), 544 (12.3);
$^1$H NMR (CH$_3$CN, 600 MHz): **1-trans** $\delta$ = 6.78-6.80 (t, $J$ = 7.6 Hz, 1H, H-7), 6.96-6.97 (br, d, $J$ = 7.0 Hz, 1H, H-3), 7.04-7.05 (d, $J$ = 7.9 Hz, 1H, H-8), 7.07 (br, 1H, H-5), 7.17-7.18 (d, $J$ = 7.6 Hz, 1H, H-6), 7.34 (br, 1H, H-4), 7.84-7.86 (dt, $J$ = 7.9 and 1.1 Hz, 1H, H-11), 7.87-7.89 (dd, $J$ = 7.9 and 1.1 Hz 1H, H-9), 7.95-7.98 (dt, $J$ = 7.9 and 1.1 Hz, 1H, H-10), 8.30-8.31 (d, $J$ = 7.6 Hz, 1H, H-12), 8.85 (br, 1H, H-2), 9.00 (br, 1H, H-1), 9.09 (br, 2H, H-15 and H-16), 9.16 (br, 6H, H-13, H-14, and H-17-20) ppm;
1-cis $\delta$ = 3.47 (br, 1H, H-6), 6.63 (s, 1H, H-7), 7.18 (br, 1H, H-4), 7.90 (s, 1H, H-8), 8.48 (s, 1H, H-9), 8.83 (br, 2H, H-10 and H-5), 9.31 (br, 1H, H-11), 9.60 (br, 1H, H-12), 39.86 (br, 1H, H-3), 42.32 (br, 2H, H-15, 16), 43.28 and 43.35 (br, 4H, H-17-20), 43.75 (br, 2H, H-13, 14), 100.08 (br, 1H, H-2), 107.88 (br, 1H, H-1) ppm;
MS [EI, m/z] 1122.1 (60 %), 1121.2 (100 %), 1015.1 (10%), 560.7 (8%);
Zur Demonstration der Reversibilität des Schaltungsvorganges wurde folgendes Experiment gemacht: Um die Langzeitstabilität und die Reversibilität des Azopyridin-funktionalisierten Ni-Porphyrins bezüglich der Schaltung mit Licht zu untersuchen wurde sowohl ein NMR-Röhrchen, als auch eine UV-Küvette abwechselnd mit Licht der Wellenlänge 500 nm und 435 nm bestrahlt. Das NMR Röhrchen enthielt 0,5 ml einer 50 $\mu$m Lösung des Porphyrins 1 in CD$_3$CN und die UV-Küvette 3 ml einer 5 $\mu$m Lösung von Porphyrin 1 in CH$_3$CN. Als Lichtquellen diente eine LEDs mit einem Emissionsmaximum bei 500 nm und einer Lichtstärke 44 lm und eine LED mit einem Emissionmaximum bei 435 nm und 35m lm. Belichtet wurde abwechseln 90 s aus einer Entfernung von 1cm mit 500 nm und 30 s mit 435 nm aus einer Entfernung von 4 cm. Bei der Belichtung mit 500 nm wurden nach 90 s 50% der Moleküle in die *cis*-Konfiguration geschaltet (50 % *trans*) und beim Belichten mit 435 nm wurden nach 30 s 90 % *trans* und 10 % *cis* Verbindung gebildet. Nach mehr als 10.000 Schaltzyklen wurden weder NMR- noch UV-spektroskopisch Nebenprodukte oder eine nachlassende Schaltfähigkeit beobachtet.

Beim Azopyridin-funktionalisierten Ni-Porphyrins entstehen auch wenn man beliebig lange mit 500 nm belichtet nicht mehr als 65 % der cis Verbindung (photostationäres Gleichgewicht). Beim Belichten mit 435 nm entsteht (wenn man lange genug belichtet) 100 % trans. Hier liegt das photostationäre Gleichgewicht vollständig auf der trans Seite.

Bezugszeichenliste

1  Chelat-Ligand
2  Uebergangsmetallion
3  quadratisch planarer oder quadratisch bipyramidaler Übergangsmetallkomplex
4  Henkel
5  axialer Ligand
6  Photochromes System
7  Koordinationsstelle im Chelat-Ligand

**Patentansprüche**

1.  Photosensitiver molekularer Schalter, mit

    - einem Chelat-Liganden,
    - einem koordinativ am Chelat-Liganden gebundenen Metallion, wobei das Metallion ausgewählt ist aus der Gruppe von Metallionen bestehend aus Mn$^{2+}$, Mn$^{3+}$, Fe$^{2+}$, Fe$^{3+}$, Co$^{2+}$ und Ni$^{2+}$,
    - einem kovalent am Chelat-Liganden gebundenen, durch Bestrahlung isomerisierbaren photochromen System, das in der einen Konfiguration am Metallion koordinativ gebunden und in der anderen Konfiguration am Metallion nicht gebunden ist.

2.  Photosensitiver molekularer Schalter nach Anspruch 1, **dadurch gekennzeichnet, dass** durch die Isomerisierung des photochromen Systems eine Änderung des magnetischen Zustandes eintritt.

3.  Photo sensitiver molekularer Schalter nach einem der vorhergehenden Ansprüche, **dadurch gekennzeichnet, dass** der Chelat-Ligand ausgewählt ist aus der Gruppe von Liganden bestehend aus Porphyrin, Phthalocyanin, Porphyrazin, Naphthocyanin, Chlorin, Bakteriochlorin, Corrin, Corrol, Salen, Glyoxim, Triethylentetramin, Cyclam-(1,4,8,11-Tetraazacyclotetradecan) und 1,4,8,11-Tetrathiocyclotetradecan-Derivaten.

4.  Photosensitiver molekularer Schalter nach einem der vorhergehenden Ansprüche, **dadurch gekennzeichnet, dass** das photochrome System Azobenzol, Phenylazopyridin oder Azopyridin, Thioindigo, Hemithioindigo, Spiropyran, Spiroindolizin, Diarylethen oder Fulgid ist.

5.  Photosensitiver molekularer Schalter nach einem der vorhergehenden Ansprüche, **dadurch gekennzeichnet, dass** der Abstand zwischen dem Metallion und dem photochromen System in der einen Konfiguration kleiner als 2,4 Å und in der anderen Konfiguration größer als 2,4 Å ist.

6.  Photosensitiver molekularer Schalter nach einem der vorhergehenden Ansprüche, **dadurch gekennzeichnet, dass** das photochrome System bei Bestrahlung mit Licht mit einer Wellenlänge von 300 bis 900 nm isomerisierbar ist.

7.  Fluid aufweisend eine Mehrzahl von als in Lösung vorliegenden Einzelmolekülen des photosensitiven molekularen Schalters nach einem der vorhergehenden Ansprüche.

8.  Verwendung eines photosensitiven Schalters nach einem der vorhergehenden Ansprüche zur berührungslosen, lichtgesteuerten Bewegung eines Objekts in einem Magnetfeld.

9.  Kontrastmittel für die Magnetresonanztomographie, aufweisend einen photosensitiven molekularen Schalter nach einem der Ansprüche 1 bis 6.

10. Speichermedium zum Speichern von Daten, aufweisend einen photosensitiven molekularen Schalter nach einem der Ansprüche 1 bis 6.

**Claims**

1.  A photosensitive molecular switch, having

    - a chelate ligand,
    - a metal ion coordinatively bound to the chelate ligand, the metal ion being selected from the group of metal ions consisting of $Mn^{2+}$, $Mn^{3+}$, $Fe^{3+}$, $Co^{2+}$, $Ni^{2+}$,

- a photochromic system that is covalently bound to the chelate ligand and can be isomerized by irradiation, this system being coordinatively bound to the metal ion in one configuration and not bound to the metal ion in the other configuration.

2. The photosensitive molecular switch according to claim 1, **characterized in that** a change of the magnetic state occurs by said isomerization of the photochromic system.

3. The photosensitive molecular switch according to one of the preceding claims, **characterized in that** the chelate ligand is selected from the group of ligands consisting of porphyrin, phthalocyanine, porphyrazine, naphthocyanine, chlorin, bacteriochlorin, corrin, corrole, salen, glyoxime, triethylenetetramine, cyclam (1,4,8,11-tetraazacyclotetradecane) and derivatives of 1,4,8,11-tetrathiocyclotetradecane.

4. The photosensitive molecular switch according to one of the preceding claims, **characterized in that** the photochromic system is azobenzene, phenylazopyridine, azopyridine, thioindigo, hemithioindigo, spiropyran, spiroindolizine, diarylethene or fulgide.

5. The photosensitive molecular switch according to one of the preceding claims, **characterized in that** the distance between the metal ion and the photochromic system is shorter than 2.4 Å in one configuration and greater than 2.4 Å in the other configuration.

6. The photosensitive molecular switch according to one of the preceding claims, **characterized in that** the photochromic system can be isomerized by irradiation with light having a wavelength of 300 to 900 nm.

7. A fluid, comprising a plurality of single molecules of the photosensitive molecular switch according to one of the preceding claims in a solution.

8. Use of a photosensitive switch according to one of the preceding claims for contactless, light-controlled movement of an object in a magnetic field.

9. A contrast agent for magnetic resonance imaging, having a photosensitive molecular switch according to one of the claims 1-6.

10. A storage medium for storing data, the storage medium having a photosensitive molecular switch according to one of the claims 1-6.

**Revendications**

1. Commutateur moléculaire photosensible, comprenant

   - un ligand chélate,
   - un ion métallique lié par coordination au ligand chélate, le liant métallique étant choisi dans le groupe des ions métalliques consistant en $Mn^{2+}$, $Mn^{3+}$, $Fe^{2+}$, $Fe^{3+}$, $Co^{2+}$ et $Ni^{2+}$,
   - un système photochrome lié par liaison covalente au ligand chélate, isomérisable par rayonnement, qui dans une configuration est lié par coordination à l'ion métallique et dans l'autre configuration n'est pas lié à l'ion métallique.

2. Commutateur moléculaire photosensible selon la revendication 1, **caractérisé en ce que** l'isomérisation du système photochrome provoque une modification de l'état magnétique.

3. Commutateur moléculaire photosensible selon l'une des revendications précédentes, **caractérisé en ce que** le ligand chélate est choisi dans le groupe des ligands consistant en la porphyrine, la phtalocyanine, la porphyrazine, la naphtocyanine, la chlorine, la bactériochlorine, la corrine, le corrol, le salène, la glycoxime, la triéthylènetétramine, le cyclame-(1,4,8-11-tétrazacyclotétradécane) et les dérivés du 1,4,8,11-tétrathiocyclotétradécane.

4. Commutateur moléculaire photosensible selon l'une quelconque des revendications précédentes, **caractérisé en ce que** le système photochrome est l'azobenzène, la phénylazopyridine ou l'azopyridine, le thioindigo, l'hémithioindigo, le spiropyranne, la spiroindolizine, le diaryléthène ou le fulgide.

5. Commutateur moléculaire photosensible selon l'une des revendications précédentes, **caractérisé en ce que** la distance entre l'ion métallique et le système photochrome est, dans l'une des configurations, inférieure à 2,4 Å, et dans l'autre configuration est supérieure à 2,4 Å.

6. Commutateur moléculaire photosensible selon l'une quelconque des revendications précédentes, **caractérisé en ce que** le système photochrome est isomérisable par irradiation avec une lumière ayant une longueur d'onde de 300 à 900 nm.

7. Fluide comprenant un grand nombre de molécules individuelles, présentes en solution, du commutateur moléculaire photosensible selon l'une quelconque des revendications précédentes.

**8.** Utilisation d'un commutateur photosensible selon l'une quelconque des revendications précédentes pour assurer un déplacement sans contact, commandé par la lumière, d'un objet dans un champ magnétique.

**9.** Produit de contraste pour tomographie par résonance nucléaire, comprenant un commutateur moléculaire photosensible selon l'une quelconque des revendications 1 à 6.

**10.** Support d'enregistrement pour l'enregistrement de données, comprenant un commutateur moléculaire photosensible selon l'une quelconque des revendications 1 à 6.

**FIG. 1**

low-spin
diamagnetisch

hν (520 nm)
hν (425 nm)
Δ

$R = C_6F_5$

high-spin
paramagnetisch

**FIG. 2**

FIG. 3

FIG. 4

**FIG. 5**

**FIG. 6**

**EP 2 605 774 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070218010 A1
- EP 2053049 A1

**In der Beschreibung aufgeführte Nicht-Patentliteratur**

- **C. BORNHOLDT.** Ligandgetriebener lichtinduzierter Spin-Crossover in Einzelmolekülen bei Raumtemperatur. *Dissertation,* 2008
- **S. KAWATA ; Y. KAWATA.** *Chem. Rev.,* 2000, vol. 100, 1777
- **U. ANDRES.** Magnetohydrodynamic and Magnetohydrostatic Methods of Mineral Separation. Wiley, 1976
- **K. A. MIRICA ; S. S. SHEVKOPLYAS ; S.T. PHILLIPS ; M. GUPTA ; G. M. WHITESIDES.** *J. Am. Chem. Soc.,* 2009, vol. 131, 10049-10058